# EUROPEAN PATENT APPLICATION

(11) **EP 1 980 257 A1**
(43) Date of publication of application: **15.10.2008**
(21) Application number: 08010897.0
(22) Date of filing: 07.04.2004
(51) Int. Cl.: A61K 31/70

(54) **Compositions and uses of galectin antagonists**

(30) Priority: 07.04.2003 US 461006 P; 07.04.2003 US 408723; 30.05.2003 US 474562 P
(62) Divisional of application: 04759200.1
(71) Applicant: Prospect Therapeutics, Inc., Woburn MA 01801 (US)
(72) Inventor: Chang, Yan, Ashland, MA 01721 (US); Sasak, Vodek, Northboro, MA 01532 (US)
(74) Representative: Chapman, Paul Gilmour

(57) **Abstract**

The present invention is directed to methods and compositions for augmenting treatment of cancers and other proliferative disorders. In particular embodiments, the invention combines the administration of an agent that inhibits the anti-apoptotic activity of galectin-3 (e.g., a "galectin-3 inhibitor") so as to potentiate the toxicity of a chemotherapeutic agent. In certain preferred embodiments, the conjoint therapies of the present invention can be used to improve the efficacy of those chemotherapeutic agents whose cytotoxicity is influenced by the status of an anti-apoptotic Bcl-2 protein for the treated cell. For instance, galectin-3 inhibitors can be administered in combination with a chemotherapeutic agent that interferes with DNA replication fidelity or cell-cycle progression of cells undergoing unwanted proliferation.

## Description

### BACKGROUND OF THE INVENTION

Galectins comprise a family of proteins which are expressed by plant and animal cells and which bind β-galactoside sugars. These proteins can be found on cell surfaces, in cytoplasm, in the nucleus, and in extracellular fluids. The two most studied galectins, galectin-1 and galectin 3, have a molecular weight in the general range of 13 - 16 kDa and 29 - 35 kD, respectively; they have an affinity for β-galactoside containing materials, and have been found to play a number of important roles in biological processes including cell migration, cell-cell adhesion, angiogenesis, cell fusion and other cell-cell interactions, as well as immune-based reactions and apoptosis. As such, the role of galectins is very strongly tied to cancer and other proliferative diseases. While there are a large number of galectins which manifest the foregoing activities, galectin-3 and galectxn-1 have been strongly implicated in connection with cellular processes involving cancers.

Galectin-3 is a carbohydrate binding protein having a molecular weight of approximately 30,000. It is composed of two distinct structural motifs, an amino-terminal portion containing Gly-X-Y tandem repeats which are characteristic of collagens, and a caiboxyl-termmal portion containing a carbohydrate binding site.

Galectin-3 is found in almost all tumors, and has a binding affinity for β - galactoside-containing glyco-conjugates. Galectin-3 is believed to play a role in mediating cell-cell interactions and thereby fostering cell adhesion, cell migration and metastatic spread. It has been found that cells which have high expressions of galectin-3 are more prone to metastasis and are more resistant to apoptosis induced by chemotherapy or radiation. It has also been reported in the literature that galectin-3 plays a role in promoting angiogenesis.

It has been shown that galectin-3 shares the "death suppression motif" of Bcl-2, a protein involved in the regulation of apoptosis, or programmed cell death. Bcl-2 is a member of a family of proteins regulating apoptosis. Some members of the family promote apoptosis, whereas others, including Bcl-2 and Bcl-xL, counterbalance by preventing it In chemoresistant cells, changes in the activities of Bcl family of proteins by changes in Bcl-2 and/or Bcl-xL expression levels, phosphorylation state, or intracellular localization, that prevent the induction of apoptosis are often implicated as the mechanism of such resistance. Inhibition of Bcl-2, Bcl-xL and related protein, in combination with the administration of cytotoxic chemotherapeutic agents, may overcome chemoresistance and restore or enhance the efficacy of chemotherapeutic agents. Overabundance of Bcl-2 and/or Bcl-xL, which is seen in some cancerous cells, correlates with the lack of cellular response to apoptosis inducers, Galectin-3 has the ability to form a heterodimer with Bcl-2, and, through this interaction, perhaps participate in the anti-apoptotic effect of Bcl-2. There is also evidence that the signal transduction pathway for galectin-3 may share some commonality with Bcl-2 pathway.

The Bcl-2 pathway is a target of many cancer treatment regimens. Neoplasts that develop or possess resistance to antineoplastic agents often have elevated levels of Bcl-2 protein and are resistant to apoptosis induction by these agents. In such instances, combination of antineoplastic agents with therapeutic agents that abolish the Hcl-2-mediated anti-apoptotic effect is an effective treatment for those patients that fail to respond to the antineoplastic agents alone.

### BRIEF SUMMARY OF THE INVENTION

One aspect of the invention provides a method for reducing the rate of growth of tumor cells or other unwanted proliferating cells related to hyperproliferative disorders such as psoriasis, rheumatoid arthritis, lamellar ichthyosis, epidermolytic hyperkeratosis, restenosis, endometriosis, abnormal wound healing, benign hyperplasias, or diseases associated with corneal neovascularization, in a patient by administering a combinatorial treatment regimen that includes:
a chemotherapeutic agent whose cytotoxicity is influenced by the status of an anti-apoptotic Bcl-2 protein for the tumor cell; and
an agent that inhibits anti-apoptotic effects of galectin-3 (herein a "galectin-3 inhibitor"), e.g., in an amount sufficient to reduce the levels of one or more G1/S cyclins in the tumor cells.

Another aspect of the invention provides a method for enhancing the pro-apoptotic effect of a chemotherapeutic agent that interferes with DNA replication fidelity or cell-cycle progression of cells undergoing unwanted proliferation, by the conjoint administration of a galectiua-3 inhibitor, e.g., in an amount sufficient to reduce the levels of one or more G1/S cyclins in the treated cells.

Still another aspect of the invention provides a method for reducing the rate of growth of tumor cells which express galectin-3 comprising, (i) obtaining a sample of tumor cells from a patient; (ii) ascertaining the galectin-3 status of the tumor cell sample; and (iii) for patients having tumor cells that express galectin-3, administering a treatment regimen including a galectin-3 inhibitor, *e.g.,* in an amount sufficient to reduce the levels of one or more G1/S cyclins in the tumor cells.

In certain preferred embodiments, the treatment regimen includes a chemotherapeutic agent that is influenced by the Bcl-2 or Bcl-xL status of the tumor cell for cytotoxicity.

Exemplary galectin-3 inhibitors include carbohydrates, antibodies, small organic molecules, peptides or polypeptides. In certain preferred embodiments, the galectin-3 inhibitor inhibits interaction of galectin-3 with an anti-apoptotic Bcl-2 protein, such as Bcl-2 or bcl-xL. In certain preferred embodiments, the inhibitor inhibits phosphorylation of galectin-3, *e.g.,* inhibits phosphorylation of galectin-3 at Ser-6. In certain preferred embodiments, the galectin-3 inhibitor inhibits translocation of galectin-3 between the nucleus and cytoplasm or inhibits galectin-3 translocation to the perinuclear membranes and inhibits cytochrome C release from mitochondria. In certain preferred embodiments, the galectin-3 inhibitor inhibits expression of galectin-3. For instance, the galectin-3 inhibitor can be an antisense or RNAi construct having a sequence corresponding to a portion of the mRNA sequence transcribed from the galectin-3 gene.

In certain preferred embodiments, the galectin-3 inhibitor is administered conjointly with a chemotherapeutic agent that induces mitochondrial dysfunction and/or caspase activation. For instance, the chemotherapeutic agent with which the galectin-3 inhibitor is administered can be one which induces cell cycle arrest at G2/M in the absence of said galectin-3 inhibitor.

Merely to illustrate, the chemotherapeutic can be an inhibitor of chromatin function, a DNA topoisomerase inhibitor, a microtubule inhibiting drug, a DNA damaging agent, an antimetabolite (such as folate antagonists, pyrimidine analogs, purine analogs, and sugar-modified analogs), a DNA synthesis inhibitor, a DNA interactive agent (such as an intercalating agent), a DNA repair inhibitor, a poly(ADF-ribose) polymerase inhibitor, an antimitotic agent, a cell cycle inhibitor, an anti-angiogonic agent, an anti-migratory agent, a differentiation modulator, a growth factor inhibitor, a hormone analog, an apoptosis inducer, a retinoic acid receptor alpha/beta selective agonist, and/or an antibiotic. In addition to conventional chemothetapeutics, the agent of the subject method can also be antisense RNA, RNAi or other polynucleotides to inhibit the expression of the cellular components that contribute to unwanted cellular proliferation that are targets of conventional chemotherapy.

In other embodiments, the subject method combines a galectin-3 inhibitor with a corticosteroid, such as cortisone, dexamethasone, hydrocortisone, methylprednisolone, prednisone, and prenisolone.

In yet other embodiments, the subject method combines a galectin-3 inhibitor with ionizing radiation.

Another aspect of the invention provides a kit that includes (i) a chemotherapeutic agent that interferes with DNA replication fidelity or cell-cycle progression of cells undergoing unwanted proliferation, (ii) a therapeutically effective amount of a galectin-3 inhibitor; and (iii) instructions and/or a label for conjoint administration of the chemotherapeutic agent and the galectin-3 inhibitor.

Still another aspect provides a packaged pharmaceutical including (i) a therapeutically effective amount of a galectin-3 inhibitor; and (ii) instructions and/or a label for administration of the galectin-3 inhibitor for the treatment of patients having tumors that that express galectin-3.

A preferred class of galectin-3 inhibitors to be used in the method of the present invention comprises a polymeric backbone having side chains dependent therefrom.

The side chains are terminated by a galactose, rhamnose, xylose, or arabinose unit. This material may be synthetic, natural, or semi-synthetic. In one particular embodiment, the therapeutic compound comprises a substantially demethoxylated polygalacturonic acid backbone which may be interrupted with rhamnose residues. Such compounds may be prepared from naturally occurring pectin, and are referred to as partially depolymerized pectin or modified pectin.

The method of present invention may be administering such materials orally, by injection, transdermally, subcutaneously or by topical application, depending upon the specific type of cancer or hyperproliferative disorder being treated, and the adjunct therapy.

### BRIEF DESCRIPTION OF THE FIGURES

Figures 1A-1C depict the promotion of apoptosis *in vitro* by formulations comprising modified pectin GCS-100 in a dose- and time-dependent manner.
Figure 2 depicts the enhancement of the efficacy of etoposide at various dosage by modified pectin GCS-100.

### DETAILED DESCRIPTION OF THE INVENTION

### I. Overview

Many chemotherapeutic agents are cytotoxic, and their effectiveness in treating cancer is based upon the fact that cancerous cells are generally more sensitive to such cytotoxic therapies than are normal cells either because of their rapid metabolism, the rate of proliferation or because they employ biochemical pathways not employed by normal cells. For many chemotherapeutics, cytotoxic effects are thought to be the consequence of inducing programmed cell death, also referred to as apoptosis. However, a major obstacle in chemotherapy can be the development of chemoresistance, which reduces or negates the effectiveness of many chemotherapeutic agents. Such resistance is often linked to the inability of the chemotherapeutic agents to induce apoptosis in particular cancer cells.

Counteracting chemoresistance can restore efficacy of many chemotherapeutic agents, and can help lower the dosage of these agents, thereby alleviating or avoiding unwanted side effects of these agents. Chemoresistance has, in several instances, been linked to alterations in anti-apoptotic Bcl-2 proteins and their pathways.

A salient feature of certain aspects of the present invention relies on a relationship between anti-apoptotic Bcl-2 proteins and galectin-3 in regulating cell death, particularly that galactin-3 has a positive effect on the apoptotic activity of these proteins. To further illustrate, galectin-3 expression has been implicated in sensitivity of tumor cells to certain chemotherapeutic agents, such as cisplatin and genistein. For instance, it has been observed that genistein effectively induces apoptosis in BT549 cells, a human breast epithelial cell line that does not express detectable levels of galectin-3. When galectin-3 transfected BT549 cells are treated with genistein, cell cycle arrest at the G(2)/M phase takes place without apoptosis induction. However, treatment of those cells with a galectin-3 inhibitor is sufficient to restore chemotherapeutic sensitivity.

The present invention is directed to methods and compositions for augmenting treatment of cancers and other hyperproliferative disorders such as psoriasis, rheumatoid arthritis, lamellar ichthyosis, epidermolytic hyperkeratosis, restenosis; endometriosis, or abnormal wound healing. The present invention provides methods of treatment of such diseases and conditions wherein administration of chemotherapeutic agents, radiation therapy, or surgery is combined with the administration of an agent that inhibits the anti-agoptotic activity of galectin-3 (e.g., a "galectin-3 inhibitor"). In particular embodiments, the combination is provided to potentiate the toxicity of a chemotherapeutic agent. In certain preferred embodiments, the conjoint therapies of the present invention can be used to improve the efficacy of those chemotherapeutic agents whose cytotoxicity is influenced by the status of an anti-apoptotic Bcl-2 protein for the treated cell. For instance, galectin-3 inhibitors can be administered in combination with a chemotherapeutic agent that interferes with DNA replication fidelity or cell-cycle progression of cells undergoing unwanted proliferation.

Moreover, it has been shown that gatectin-3 induces cyclin D(1) promoter activity in certain tumor cells. C.f, Lin et al., 2002, Oncogene 21:8001-10. D-type cyclins coordinate cell cycle activation by regulating cyclin D-dependent kinases ("cdk"), and they are essential for the progression through the G1 phase of the cell cycle.

This pathway is known to be deregulated in a large number of human neoplasms. It has also been postulated that overexpression of cyclin D, which shortens the duration of the G1 transition, results in mild radiation resistance in breast cancer, perhaps by inhibiting apoptosis. Xia et al., 2002, Semin. Radiat. Oncol. 12:296-304. In addition, the status of anti-apoptotic Bcl-2 proteins can also influence the efficacy of killing by radiation. Thus, another aspect of the present relates to reducing tolerance to radiation therapy by administering a galectin-3 inhibitor.

Through the methods of the present invention, the dosages of potentially toxic therapies such as chemotherapies and radiation may be reduced and chemoresistance may be overcome. These and other advantages of the invention will be discussed herein below.

The present invention also provides treatment programs in which the galectin-3 status of a diseased cell sample is ascertained, and for patients having unwanted proliferating cells that express galectin-3, a treatment regimen is instituted that includes a galectin-3 inhibitor.

Another aspect of the invention relies on the observation that galectins are involved in promoting angiogenesis. In order for a solid tumor to grow or metastasize the tumor must be vascularized. Galectin-3 in particular has been demonstrated to affect chemotaxis and morphology, and to stimulate angiogenesis *in vivo.* In accord with the present invention, a galectin inhibitor is administered to a patient in combination with conventional chemotherapy.

Depending on the nature of the cancer and the therapy, the galectin inhibitor may be administered prior to, contemporaneously with and/or after other therapies. When administration contemporaneously with other drugs, the galectin inhibitor may be formulated separately from, or co-formulated with, one or more of the other drugs.

### II. Definitions

The terms "apoptosis" or "programmed cell death," refers to the physiological process by which unwanted or useless cells are eliminated during development and other normal biological processes. Apoptosis, is a mode of cell death that occurs under normal physiological conditions and the cell is an active participant in its own demise ("cellular suicide"). It is most often found during normal cell turnover and tissue homeostasis, embryogenesis, induction and maintenance of immune tolerance, development of the nervous system and endocrine-dependent tissue atrophy. Cells undergoing apoptosis show characteristic morphological and biochemical features.

These features include chromatin aggregation, nuclear and cytoplasmic condensation, partition of cytoplasm and nucleus into membrane bound vesicles (apoptotic bodies) which contain ribosomes, morphologically intact mitochondria and nuclear material. Cytochrome C release from mitochondria is seen as an indication of mitochondrial dysfunction accompanying apoptosis. *In vivo,* these apoptotic bodies are rapidly recognized and phagocytized by either macrophages or adjacent epithelial cells. Due to this efficient mechanism for the removal of apoptotic cells *in vivo* no inflammatory response is elicited. *In vitro,* the apoptotic bodies as well as the remaining cell fragments ultimately swell and finally lyse. This terminal phase of *in vitro* cell death has been termed "secondary necrosis."

The term "anti-apoptotic Bcl-2 protein" refers to a family of proteins related to the Bcl-2 protein and which are antagonists of cellular apoptosis. This family includes Bcl-2, Bcl-xL, Bcl-w, Mcl-1 and A-1. See, for example, Hockenbery et al., 1990, Nature 348:334-336; Boise et al., 1993, Cell 74.-597-608; Gibson et al., 1996, Oncogene 13:665-675; Zhou et al., 1997, Blood 89:630-643; and Lin et al., 1993, J Immunol. 151:1979-1988. This family of proteins shares four homology regions, termed Bcl homology (BH) domains, namely BH1, BH2, BH3, and BH4. A representative sequence for a human Bcl-2 coding sequence and protein are provided in GenBank Accession NM_000657 (GI 4557356). A representative sequence for a human Bcl-xL coding sequence and protein are provided in GenBank Accession Z23115 (GI 510900). Exemplary anti-apoptotic Bcl-2 proteins are those which are at least 90 percent identical to the protein sequences set forth in GenBank Accessions NM_000657 or Z23115, and/or which can be encoded by a nucleic acid sequence that hybridizes winder stringent wash conditions of 0.2 x SSC at 65C to a coding sequence set forth in GenBank Accessions NM_000657 or Z23115.

The term "status of anti-apoptotic Bcl-2 proteins" includes within its meaning such quantitative measurement as: the level of mRNA encoding an anti-apoptotic Bcl-2 protein; the level of the protein; the number and location o4 or the absence of, phosphorylated residues or other posttranslational modifications of the protein; the intracellular localization of the protein; the status of association of anti-apoptotic Bcl-2 proteins with each other or with other proteins; and/or any other surrogate or direct measurement of anti-apoptotic activity due to an anti-apoptotic Bcl-2 protein.

More specifically, the term "status of anti-apoptotic Bcl-2 protein levels" means the amount of anti-apoptotic Bcl-2 proteins in a cell, such as may be detected by immunohistochemistry using antibodies specific to an anti-apoptotic Bcl-2 protein.

As used herein the term "animal" refers to mammals, preferably mammals such as humans, Likewise, a "patient" or "subject" to be treated by the method of the invention can mean either a human or non-human animal.

The term "antibody" as used herein, unless indicated otherwise, is used broadly to refer to both antibody molecules and a variety of antibody-derived molecules. Such antibody derived molecules comprise at least one variable region (either a heavy chain of light chain variable region), as well as individual antibody light chains, individual antibody heavy chains, chimeric fusions between antibody chains and other molecules, and the like. Functional immunoglobulin fragments according to the present invention may be Fv, scFv, disulfide-linked Fv, Fab, and F(ab')₂.

As used herein, the term "cancer" refers to any neoplastic disorder, including such cellular disorders as, for example, renal cell cancer, Kaposi's sarcoma, chronic leukemia, prostate cancer, breast cancer, sarcoma, pancreatic cancer, ovarian carcinoma, rectal cancer, throat cancer, melanoma, colon cancer, bladder cancer, mastocytoma, lung cancer, mammary adenocarcinoma, myeloma, lymphoma, pharyngeal squamous cell carcinoma, and gastrointestinal or stomach cancer. Preferably, the cancer which is treated in the present invention is melanoma, lung cancer, breast cancer, pancreatic cancer, prostate cancer, colon cancer, or ovarian cancer.

The "growth state" of a cell refers to the rate of proliferation of the cell and the state of differentiation of the cell.

As used herein, "hyperproliferative disease" or "hyperproliferative disorder" refers to any disorder which is caused by or is manifested by unwanted proliferation of cells in a patient. Hyperproliferative disorders include but are not limited to cancer, psoriasis, rheumatoid arthritis, lamellar ichthyosis, epidermolytic hyperkeratosis, restenosis, endometriosis, and abnormal wound healing.

As used herein, "proliferating" and "proliferation" refer to cells undergoing mitosis.

As used herein, "unwanted proliferation" means cell division and growth that is not part of normal cellular turnover, metabolism, growth, or propagation of the whole organism. Unwanted proliferation of cells is seen in tumors and other pathological proliferation of cells, does not serve normal function, and for the most part will continue unbridled at a growth rate exceeding that of cells of a normal tissue in the absence of outside intervention. A pathological state that ensues because of the unwanted proliferation of cells is referred herein as a "hyperproliferative disease" or "hyperproliferative disorder."

As used herein, "transformed cells" refers to cells that have spontaneously converted to a state of unrestrained growth, i.e., they have acquired the ability to grow through an indefinite number of divisions in culture. Transformed cells may be characterized by such terms as neoplastic, anaplastic and/or hyperplastic, with respect to their loss of growth control For purposes of this invention, the terms "transformed phenotype of malignant mammalian cells" and "transformed phenotype " are intended to encompass, but not be limited to, any of the following phenotypic traits associated with cellular transformation of mammalian cells: immortalization, morphological or growth transformation, and tumorigenicity, as detected by prolonged growth in cell culture, growth in semi-solid media, or tumorigenic growth in immuno-incompetent or syngeneic animals.

### III. Exemplary Embodiments

### A. Galectin-3 Inhibitors

In certain embodiments of the present invention, the galectin-3 inhibitor is an agent that binds to galoctin-3 and reduces its anti-apoptotic activity. Such agents can work, for example, by preventing intracellular signal transduction pathways and/or translocation of gamectin-3. Merely to illustrate, the agent can be one which inhibits, the multimerization of galectin-3 and/or its interaction of galectin-3 with an anti-apoptotic Bcl-2 protein, such as Bcl-2 or bel-xL. It may also be an agent that inhibits phosphorylation of galectin-3, such as by inhibiting phosphorylation of galectin-3 at Ser-6. At a gross mechanistic level, the inhibitor can be an agent that inhibits translocation of galectin-3 between the nucleus and cytoplasm or inhibits galectin-3 translocation to the perinuclear membranes and inhibits cytochrome C release from mitochondria.

One class of galectin-3 inhibitors contemplated by the present invention are polymers, particularly carbohydrate containing polymers, that bind to galectin-3 and inhibit its anti-apoptotic activity. Materials useful in the present inventions may be generally comprised of natural or synthetic polymers and oligomers. Preferably, such polymers are very low in toxicity and interact synergistically with heretofore employed cancer therapies so as to increase the effectiveness thereof.

A preferred class of polymers for the practice of the present invention are carbohydrate-derived polymers which contain an active galectin binding sugar site, but which have somewhat higher molecular weights than simple sugars so that such molecules are capable of sustained blocking, activating, suppressing, or otherwise interacting with other portions of the galectin protein. A preferred class of therapeutic materials comprises oligomeric or polymeric species of natural or synthetic origin, rich in galactose or arabinose. Such materials will preferably have a molecular weight in the range of up to 500,000 daltons and, more preferably, in the range of up to 100,000 daltons. One particular material comprises a substantially demethoxylated polygalacturonic acid backbone which may be interrupted by rhamnose with galactose terminated side chains pendent therefrom. Another particular material comprises a homogalacturonan backbone with or without side chains pendent therefrom.

One group of materials falling within, this general class comprises a substantially demethoxylated polygalacturonic acid backbone having rhamnose, galactose, arabinose or other sugar residues pendent therefrom. It is believed that in materials of this type, the terminal galactose or arabinose units pendent from the backbone bind to galectin proteins. The remaining bulk of the molecule potentiates the compound's action in moderating immune system response. Materials of this general type are described by formulas I and II below, and it is to be understood that yet other variants of this general compound may be prepared and utilized in accord with the principles of the present invention.
Homogalacturonan

- [α-Gal*p*A-(1→4)-α-Gal*p*A]ₙ- (I)

Rhamnogalacturonan

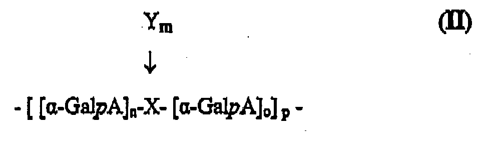

In the formulae above, m is ≥ 0, n, o and p are ≥1, X is α-Rhap; and Ym represents a linear or branched chain of sugars (each Y in the chain Ym can independently represent a different sugar within the chain). The sugar Y may be, but is not limited to, any of the following α-Gal*p,* β-Gal*p,* β-Api*f,* β-Rha*p*, α-Rha*p* , α-Fuc*p,* β-GIc*p*A, α-Gal*p*A, β-Gal*p*A, β-Dha*p*A,. Kdo*p*, β-Ace*f*, α-Araf, β-Araf', and α-Xylp.

It will be understood that natural pectin does not possess a strictly regular repeating structure, and that additional random variations are likely to be introduced by partial hydrolysis of the pectin, so that the identity of Ym and the values ofn and o may vary from one iteration to the next of the p repeating units represented by formula II above.

Abbreviated monomer names used herein are defined as follows: GalA: galactnronic acid, Rha: rhamnose, Gal: galactose, Api: erythro-apiose, Fuc: fucose, GlcA: glucuronic acid, DhaA: 3-deoxy-D-*lyxo*-heptulosaric acid, Kdo: 3-deoxy-D-*manno*-2-octulosonic acid, Ace: aceric acid (3-C-carboxy-5-deoxy-L-lyxose), Ara: arabinose. Italicized *p* stands for pyranose and italicized *f* stands for furanose.)

An exemplary polymer of this type is modified pectin, preferably water soluble pH modified citrus pectin. Suitable polymers of this type are disclosed in, for example U.S. Patents 5,834,442, 5,895,784, 6,274,566 and 6,500,807, and PCT Publication WO 03/000118.

Pectin is a complex carbohydrate having a highly branched structure comprised of a polygalacturonic backbone with numerous branching side chains dependent therefrom. The branching creates regions which are characterized as being "smooth" and "hairy." It has been found that pectin can be modified by various chemical, enzymatic or physical treatments to break the molecule into smaller portions having a more linearized, substantially demethoxylated, polygalacturonic backbone with pendent side chains of rhamnose residues having decreased branching. The resulting partially depolymerized pectin is known in the art as modified pectin, and its efficacy in treating cancer has been established; although galectin blocker materials of this type have not been used in conjunction with surgery, chemotherapy or radiation.

U.S, Patent 5,895,784, the disclosure of which is incorporated herein by reference, describes modified pectin materials, techniques for their preparation, and use of the material as a treatment for various cancers. The material of the '784 patent is described as being prepared by a pH based modification procedure in which the pectin is put into solution and exposed to a series of programmed changes in pH which results in the breakdown of the molecule to yield therapeutically effective modified pectin. The material in the '784 patent is most preferably prepared from citrus pectin; although, it is to be understood that modified pectins may be prepared from pectin from other sources, such as apple pectin. Also, modification may be done by enzymatic treatment of the pectin, or by physical processes such as heating. Further disclosure of modified pectins and techniques for their preparation and use are also found in U.S. Patent 5,834,442 and U.S. Patent Application Serial No. 08/024,487, the disclosures of which are incorporated herein by reference. Modified pectins of this type generally have molecular weights in the range of less than 100 kilodalton. A group of such materials has an average molecular weight of less than 3 kilodalton. Another group has an average molecular weight in the range of 1-15 kilodalton, with a specific group of materials having a molecular weight of about 10 kilodalton. In one embodiment, modified pectin has the structure of a pectic acid polymer with some of the pectic side chains still present In preferred embodiments, the modified pectin is a copolymer of homogalacturonic acid and rhamnogalacturonan I in which some of the galactose- and arabinose-containing sidechains are still attached. The modified pectin may have a molecular weight of 1 to 500 kilodaltons (kD), preferably 10 to 250 kD, more preferably 50-200 kD, 70-150 kD, and most preferably 80 to 100 kD as measured by Gel Permeation Chromatography (GPC) with Multi Angle Laser Light Scattering (MALLS) detection.

Degree of esterification is another characteristic of modified pectins. In certain embodiments, the degree of esterification may be between 0 and 80%, preferably 0 to 50%, more preferably 0 to 25% and most preferably less than 10%.

Saccharide content is another characteristic of modified pectins. In certain embodiments, the modified pectin is composed entirely of a single type of saccharide subunit. In other embodiments, the modified pectin comprises at least two, preferably at least three, and most preferably at least four types of saccharide subunits. For example, the modified pectin may be composed entirely of galacturonic acid subunits. Alternatively, the modified pectin may comprise a combination of galacturonic acid and rhamnose subunits. In yet another example, the modified pectin may comprise a combination of galacturonic acid, rhamnose, and galactose subunits. In yet another example, the modified pectin may comprise a combination of galacturonic acid, rhamnose, and arabinose subunits. In still yet another example, the modified pectin may comprise a combination of galacturonic acid, rhamnose, galactose, and arabinose subunits. In some embodiments, the galacturonic acid content of modified pectin is greater than 50%, preferably greater than 60% and most preferably greater than 80%. In some embodiments, the rhamnose content is less than 25%, preferably less than 15% and most preferably less than 10%; the galactose content is less than 50%, preferably less than 40% and most preferably less than 30%; and the arabinose content is less than 15%, preferably less than 10% and most preferably less than 5%. In certain embodiments, the modified pectin may contain other uronic acids, xylose, ribose, lyxose, glucose, allose, altrose, idose, talose, gluose, mannose, fructose, psicose, sorbose or talalose in addition to the saccharide units mentioned above.

Modified pectin suitable for use in the subject methods may also have any of a variety of linkages or a combination thereof. By linkages it is meant the sites at which the individual sugars in pectin are attached to one another. In some embodiments, the modified pectin comprises only a single type of linkage. In certain preferred embodiments, the modified pectin comprises at least two types of linkages, and most preferably at least 3 types of linkages. For example, the modified pectin may comprise only alpha-1,4-linked galacturonic acid subunits. Alternatively, the modified pectin may comprise alpha-1,4-linked galacturonic acid subunits and alpha-1,2-rhamnose subunits. In another example, the modified pectin may be composed of alpha-1,4-linked galacturonic acid subunits and alpha-1,2-rhamnose subunits linked through the 4 position to arabinose subunits. In another example, the modified pectin may comprise alpha-1,4-linked galacturonic acid subunits and alpha-1,2-rhamnose subunits linked through the 4 position to arabinose subunits with additional 3-linked arabinose subunits. In another example, the modified pectin may comprise alpha-1,4-linked galacturonic acid subunits and alpha 1,2-rhamvose subunits linked through the 4 position to arabinose subunits with additional 5-linked arabinose units. In another example, the modified pectin may comprise alpha-1,4-bnked galacturonic acid subunits and alpha-1,2-zhamzzose subunits linked through the 4 position to arabinose subunits with additional 3-liked and 5-linked arabinose subunits. In another example, the modified pectin may comprise alpha-1,4-linkod galacturonic acid subunits and alpha-1,2-rhamnose subunits linked through the 4 position to arabinose subunits with additional 3-linked and 5-linked arabinose subunits with 3,5-linked arabinose branch points. In another example, the modified pectin may comprise alpha-1,4-linked galacturonic acid subunits and alpha-1,2-rhamnose subunits linked through the 4 position to galactose subunits. In another example, the modified pectin may comprise alpha-1,4-linked galacturonic acid subunits and alpha-1,2-rhamnose subunits linked through the 4 position to galactose subunits with additional 3-linked galactose subunits. In another example, the modified pectin may comprise alpha-1,4-linked galacturonic acid subunits and alpha-1,2-rhamnose subunits linked through the 4 position to galactose subunits with additional 4-linked galactose subunits. In another example, the modified pectin may comprise alpha-1,4-linked galacturonic acid subunits and alpha-1,2-rhamose subunits linked through the 4 position to galactose subunits with additional 3-linked galactose subunits with 3,6-linked branch points. In another example, the modified pectin may comprise alpha-1,4-linkcd galacturonic acid subunits and alpha-1,2-rhamnosa subunits linked through the 4 position to galactose subunits with additional 4-linked galactose subunits with 4,6-linked branch points. In certain embodiments, the side chains of the modified pectin may comprise uronic acids, galacturonic acid, glucuronic acid, rhamnose, xylose, ribose, lyxose, glucose, allose, altrose, idose, talose, gluose, mannose, fructose, psicose, sorbose or talalose in addition to the saccharide units described above.

In certain embodiments, the modified pectin preparation is a substantially ethanol-free product suitable for parenteral administration. By substantially free of ethanol, it is meant that the compositions of the invention contain less than 5% ethanol by weight In preferred embodiments the compositions contain less than 2%, and more preferably less than 0.5% ethanol by weight. In certain embodiments, the compositions further comprise one or more pharmaceutically acceptable excipients. Such compositions include aqueous solutions of the modified pectin of the invention. In certain embodiments of such aqueous solutions, the pectin modification occurs at a concentration of at least 7 mg/mL, and preferably at least 10 or even 15 or more mg/mL Any of such compositions are also substantially free of organic solvents other than ethanoL

The apoptosis-promoting activity of a modified pectin material is illustrated in Example 1, below.

Other classes of galectin-3 inhibitors that bind to galectin-3 include antibodies specific to galectin-3, peptides and polypeptides that bind to and interfere with galectin-3 activity, and small (preferably less than 2500amu) organic molecules that bind to galecttn-3.

To further illustrate, in certain embodiments of the present invention, the subject methods can be carried out using an antibody that is immunoreactive with galectin-3 and inhibitory for its anti-apoptotic activity.

An exemplary protein therapeutic is described in PCT publication WO 02/100343.

That reference discloses certain N-Terminally truncated galectin-3 proteins that inhibit the binding of intact galectin-3 to carbohydrate ligands and thereby also inhibit the multimerization and cross-linking activities of galectin-3 that may be required for its anti-apoptotic activity.

Exemplary small molecule inhibitors of galectin-3 include thiodigalactoside (such as described in Leffler et al., 1986, J. Biol. Chem. 261:10119) and agents described in PCT publication WO 02/057284.

In certain preferred embodiments of galectin-3 inhibitors that bind to galectin-3, the inhibitor is selected to having a dissociation constant (Kd) for binding galectin-3 of 10⁻⁶ M or less, and even more preferably less than 10⁻⁷ M, 10⁻⁸ M or even 10⁻⁹ M.

Certain of the galectin-3 inhibitors useful in the present invention act by binding to galectin-3 and disrupting galectin-3's interactions with one or more anti-apoptotic Bcl-2 proteins. A galectin-3 inhibitor may bind directly to the Bcl-2 binding site thereby competitively inhibits Bcl-2 binding. However, galectin-3 inhibitors which bind to the Bcl-2 protein are also contemplated, and include galectin-3 inhibitors that bind to a Bcl-2 protein and either competitively or allosterically inhibit interaction with galectin-3.

As mentioned above, certain of the subject galectin-3 inhibitors exert their effect by inhibiting phosphorylation of galectin-3. The binding of a galectin-3 inhibitor may block the access of kinases responsible for galectin-3 phosphorylation, or, alternatively, may cause conformational change of galectin, concealing or exposing the phosphorylation sites. However, the present invention also contemplates the use of kinase inhibitors which act directly on the kinase(s) that is responsible for phosphorylating galectin-3.

In still other embodiments, inhibition of galectin-3 activity is also achieved by inhibiting expression of galectin-3 protein. Such inhibition is achieved using an antisense or RNAi construct having a sequence corresponding to a portion of the mRNA sequence transcribed from the galectin-3 gene.

In certain embodiments, the galectin-3 inhibitors can be nucleic acids. In one embodiment, the invention relates to the use of antisense nucleic acid that hybridizes to the galectin-3 mRNA and decreases expression of galectin-3. Such an antisense nucleic acid can be delivered, for example, as an expression plasmid which, when transcribed in the cell, produces RNA which is complementary to at least a unique portion of the cellular mRNA which encodes galectin-3. Alternatively, the construct is an oligonucleotide which is generated *ex vivo* and which, when introduced into the cell causes inhibition of expression by hybridizing with the mRNA and/or genomic sequences encoding galectin-3. Such oligonucleotide are optionally modified oligonucleotide which are resistant to endogenous nucleases, e.g., exonucleases and/or endonucleases, and is therefore stable *in vivo.* Exemplary nucleic acid molecules for use as antisense oligonucleotides are phosphoramidate, phosphothioate and methylphosphonate analogs of DNA (see also U.S. Patent Nos. 5,176,996; 5,264,564; and 5,256,775). Additionally, general approaches to constructing oligomers useful in nucleic acid therapy have been reviewed, for example, by van der Krol et al., (1988) Biotechniques 6:958-976; and Stein et al., 1988, Cancer Res. 48:2659-2668.

In another embodiment, the invention relates to the use of RNA interference (RNAi) to effect knockdown of expression of the galectin-3 gene. RNAi constructs comprise double stranded RNA that can specifically block expression of a target gene. "RNA interference" or "RNAi" is a term initially applied to a phenomenon observed in plants and worms where double-stranded RNA (dsRNA) blocks gene expression in a specific and post-transcriptional manner. RNAi provides a useful method of inhibiting gene expression *in vitro* or *in vivo.* As used herein, the term "RNAi construct" is a generic term including small interfering RNAs (siRNAs), hairpin RNAs, and other RNA species which can be cleaved in vivo to form siRNAs. RNAi constructs herein also include expression vectors (also referred to as RNAi expression vectors) capable of giving rise to transcripts which form dsRNAs or hairpin RNAs in cells, and/or transcripts which can produce siRNAs *in vivo.*

RNAi constructs can comprise either long stretches of dsRNA identical or substantially identical to the target nucleic acid sequence or short stretches of dsRNA identical to substantially identical to only a region of the target nucleic acid sequence.

Optionally, the RNAi constructs contain a nucleotide sequence that hybridizes under physiologic conditions of the cell to the nucleotide sequence of at least a portion of the mRNA transcript for the gene to be inhibited (*i.e.,* the "target" gene). The double-stranded RNA need only be sufficiently similar to natural RNA that it has the ability to mediate RNAi. Thus, the invention has the advantage of being able to tolerate sequence variations that might be expected due to genetic mutation, strain polymorphism or evolutionary divergence. The number of tolerated nucleotide mismatches between the target sequence and the RNAi construct sequence is no more than 1 in 5 base pairs, or 1 in 10 base pairs, or 1 in 20 base pairs, or 1 in 50 base pairs. Mismatches in the center of the siRNA duplex are most critical and may essentially abolish cleavage of the target RNA. In contrast, nucleotides at the 3' end of the siRNA strand that is complementary to the target RNA do not significantly contribute to specificity of the target recognition. Sequence identity may be optimized by sequence comparison and alignment algorithms known in the art (*see* Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group). Greater than 90% sequence identity, or even 100% sequence identity, between the inhibitory RNA and the portion of the target gene is preferred. Alternatively, the duplex region of the RNA may be defined functionally as a nucleotide sequence that is capable of hybridizing with a portion of the target gene transcript (e.g., 400 mM NaCl, 40 mM PIPES pH 6.4, 1 mM EDTA, 50 °C or 70 °C hybridization for 12-16 hours; followed by washing).

The double-stranded structure may be formed by a single self-complementary RNA strand or two complementary RNA strands. RNA duplex formation may be initiated either inside or outside the cell. The RNA may be introduced in an amount which allows delivery of at least one copy per cell. Higher doses (*e.g.,* at least 5, 10, 100, 500 or 1000 copies per cell) of double-stranded material may yield more effective inhibition, while lower doses may also be useful for specific applications. Inhibition is sequence-specific in that nucleotide sequences corresponding to the duplex region of the RNA are targeted for genetic inhibition.

The subject RNAi constructs can be "small interfering RNAs" or "siRNAs." These nucleic acids are around 19-30 nucleotides in length, and even more preferably 21-23 nucleotides in length. The siRNAs are understood to recruit nuclease complexes and guide the complexes to the target mRNA by pairing to the specific sequences.

As a result, the target mRNA is degraded by the nucleases in the protein complex. In a particular embodiment, the 21-23 nucleotides siRNA molecules comprise a 3' hydroxyl group. In certain embodiments, the siRNA constructs can be generated by processing of longer double-stranded RNAs, for example, in the presence of the enzyme dicer. In one embodiment, the Drosophila *in vitro* system is used. In this embodiment, dsRNA is combined with a soluble extract derived from Drosophila embryo, thereby producing a combination. The combination is maintained under conditions in which the dsRNA is processed to RhTA molecules of about 21 to about 23 nucleotides. The siRNA molecules can be purified using a number of techniques known to those of skill in the art. For example, gel electrophoresis can be used to purify siRNAs. Alternatively, non-denaturing methods, such as non-denaturing column chromatography, can be used to purify the siRNA. In addition, chromatography (*e*.*g*., size exclusion chromatography), glycerol gradient centrifugation, affinity purification with antibody can be used to purify siRNAs.

Production of RNAi constructs can be carried out by chemical synthetic methods or by recombinant nucleic acid techniques. Endogenous RNA polymerase of the treated cell may mediate transcription *in vivo,* or cloned RNA polymerase can be used for transcription *in vitro.* The RNAi constructs may include modifications to either the phosphate-sugar backbone or the nucleoside, e.g., to reduce susceptibility to cellular nucleases, improve bioavailability, improve formulation characteristics, and/or change other pharmacokinetic properties. For example, the phosphodiester linkages of natural RNA may be modified to include at least one of an nitrogen or sulfur heteroatom. Modifications in RNA structure may be tailored to allow specific genetic inhibition while avoiding a general response to dsRNA. Likewise, bases may be modified to block the activity of adenosine deaminase. The RNAi construct may be produced enzymatically or by partial/total organic synthesis, any modified ribonucleotide can be introduced by *in vitro* enzymatic or organic synthesis.

Methods of chemically modifying RNA molecules can be adapted for modifying RNAi constructs *(see, e.g.,* Heidenreich et al., 1997, Nucleic Acids Res., 25:776-780; Wilson et al., 1994, J. Mol. Recog. 7:89-98; Chen et al., 1995, Nucleic Acids Res. 23:2661-2668; Hirschbein et al., 1997, Antisense Nucleic Acid Drug Dev. 7:55-61). Merely to illustrate, the backbone of an RNAi construct can be modified with phosphorothioates, phosphoramidate, phosphodithioates, chimeric methylphosphonate-phosphodiesters, peptide nucleic acids, 5-propynyl-pyrimidine containing oligomers or sugar modifications (*e.g.,* 2'-substituted ribonucleosides, a-configuration).

In some cases, at least one strand of the siRNA molecules has a 3' overhang from about 1 to about 6 nucleotides in length, though may be from 2 to 4 nucleotides in length. More preferably, the 3' overhangs are 1-3 nucleotides in length. In certain embodiments, one strand having a 3' overhang and the other strand being blunt-ended or also having an overhang. The length of the overhangs may be the same or different for each strand. In order to further enhance the stability of the siRNA, the 3' overhangs can be stabilized against degradation. In one embodiment, the RNA is stabilized by including purine nucleotides, such as adenosine or guanosino nucleotides. Alternatively, substitution of pyrimidine nucleotides by modified analogues, e.g., substitution of uridine nucleotide 3' overhangs by 2'-deoxythyinidine is tolerated and does not affect the efficiency of pNAi. The absence of a 2' hydroxyl significantly enhances the nuclease resistance of the overhang in tissue culture medium and may be beneficial *in vivo.*

The RNAi construct can also be in the form of a long double-stranded RNA. In certain embodiments, the RNAi construct is at least 25, 50, 100, 200, 300 or 400 bases. In certain embodiments, the RNAi construct is 400-800 bases in length. The double-stranded RNAs are digested intracellularly, e.g., to produce siRNA sequences in the cell. However, use of long double-stranded RNAs *in vivo* is not always practical, presumably because of deleterious effects which may be caused by the sequence-independent dsRNA response. In such embodiments, the use of local delivery systems and/or agents which reduce the effects of interferon or PKR are preferred.

Alternatively, the RNAi construct is in the form of a hairpin structure (named as hairpin RNA). The hairpin RNAs can be synthesized exogenously or can be formed by transcribing from RNA polymerase III promoters *in vivo.* Examples of making and using such hairpin RNAs for gene silencing in mammalian cells are described in, for example, Paddison et al., Genes Dev ., 2002, 16:948-58; McCaffrey et al., Nature, 2002, 418:38-9; McManus et al., RNA, 2002, 8:842-50; Yu et al., Proc. Nat'l Acad. Sci. USA, 2002, 99:6047-52). Preferably, such hairpin RNAs are engineered in cells or in an animal to ensure continuous and stable suppression of a desired gene. It is known in the art that siRNAs can be produced by processing a hairpin RNA in the cell.

PCT application WO 01/77350 describes an exemplary vector for bi-directional transcription of a transgene to yield both sense and antisense RNA transcripts of the same transgene in a eukaryotic cell. Accordingly, in certain embodiments, the present invention provides a recombinant vector having the following unique characteristics: it comprises a viral replicon having two overlapping transcription units arranged in an opposing orientation and flanking a transgene for an RNAi construct of interest, wherein the two overlapping transcription units yield both sense and antisense RNA transcripts from the same transgeno fragment in a host cell.

In another embodiment, the invention relates to the use of ribozyme molecules designed to catalytically cleave galectin-3 mRNA transcripts to prevent translation of mRNA (*see, e.g.,* PCT International Publication WO90/11364, published October 4, 1990; Sarver et al., 1990, Science 247:1222-1225; and U.S. Patent No. 5,093,246). While ribozymes that cleave mRNA at site-specific recognition sequences can be used to destroy particular mRNAs, the use of hammerhead ribozymes is preferred. Hammerhead ribozymes cleave mRNAs at locations dictated by flanking .regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, 1988, Nature, 334:585-591. The ribozymes of the present invention also include RNA endoribonucleases ("Cech-type ribozymes") such as the one which occurs naturally in *Tetrahymena thermophila* (known as the IVS or L-19 IVS RNA) and which has been extensively described (*see, e.g.,* Zaug, et al., 1984, Science, 224:574-578; Zaug and Cech, 1986, Science, 231:A.70-475; Zaug, et al., 1986, Nature, 324;429-433; published International patent application No. WO88/04300 by University Patents Inc.; Been and Cech, 1986, Cell, 47:207-216).

In a further embodiment the invention relates to the use of DNA enzymes to inhibit expression of the galectin-3 gene. DNA enzymes incorporate some of the mechanistic features of both antisense and ribozyme technologies. DNA enzymes are designed so that they recognize a particular target nucleic acid sequence, much like an antisense oligonucleotide, however much like a ribozyme they are catalytic and specifically cleave the target nucleic acid. Briefly, to design an ideal DNA enzyme that specifically recognizes and cleaves a target nucleic acid, one of skill in the art must first identify the unique target sequence. Preferably, the unique or substantially sequence is a G/C rich of approximately 18 to 22 nucleotides. High G/C content helps insure a stronger interaction between the DNA enzyme and the target sequence. When synthesizing the DNA enzyme, the specific antisense recognition sequence that will target the enzyme to the message is divided so that it comprises the two arms of the DNA enzyme, and the DNA enzyme loop is placed between the two specific arms. Methods of making and administering DNA enzymes can be found, for example, in U.S. Patent No. 6,110,462.

### B. Chemotherapeutic Agents

Pharmaceutical agents that may be used in the subject combination chemotherapy include, merely to illustrate: aminoglutethimide, amsacrine, anastrozole, asparaginase, beg, bicalutamide, bleomycin, buserelin, busulfan, campothecin, capecitabine, carboplatin, carmustine, chlorambucil, cisplatin, cladribine, clodronate, colchicine, cyclophosphamide, cyproterone, cytarabine, dacarbazine, dactiaomycin, daunorubicin, dienestrol, diethylstilbestrol, docetaxel, doxorubicin, epirubicin, estradiol, estramustine, etoposide, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, ironotecan, letrozole, leucovorin, leuprolide, levamisole, lomustine, mechlorethamine, medroxyprogasterone, megestrol, melphalan, mercaptopurine, mesna, methotrexate, mitomycin, mitotane, mitoxantrone, nilutamide, nocodazole, octreotide, oxaliplatin, paclitaxel, pamidronate, pentostatin, plicamycin, porfimer, procarbazine, raltitrexed, rituximab, streptozocin, suramin, tamoxifen, temozolomide, teniposide, testosterone, thioguanine, thiotepa, titanocene dichloride, topotecan, trastuzumab, tretinoin, vinblastine, vincristine, vindesine, and vinorelbine.

These chemotherapeutic agents may be categorized by their mechanism of action into, for example, following groups: anti-metabolites/anti-cancer agents, such as pyrimidine analogs (5-fluorouracil, floxuridine, capecitabine, gemcitabine and cytarabine) and purine analogs, folate antagonists and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristin, vinblastin, nocodazole, epothilones and navelbine, epidipodophyllotoxins (teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, docetaxel, doxorubicin, epirubicin, hexamethylmelamineoxaliplatin, iphosphamide, melphalan, merchlorathamine, mitomycin, mitoxantrone, nitrosourea, paclitaxel, plicamycin, procarbazine, teniposide, triethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, stroptozocin), trazcnes - dacarbazinine (DTIC); antiproliferative/antimitotic antimetabolites such as folic acid analogs (methotrexate); platinum coordination complexes (cisplatin, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, COX-2 inhibitors, dipyridamole, ticlopidine, clopidogrel, abciximab; antimigratory agents; antisecretory agents (breveldin); immunosuppressives (cyclosporine, tacrolimus (FK-506), sirolimus (rapamycin), azathioprine, mycophenolate mofetil); anti-angiogenic compounds (TNP-470, genistein) and growth factor inhibitors (vascular endothelial growth factor (VEGF) inhibitors, fibroblast growth factor (FGF) inhibitors, epidermal growth factor (EGF) inhibitors); angiotensin receptor blocker, nitric oxide donors; anti-sense oligonucleotides; antibodies (trastuzumab); cell cycle inhibitors and differentiation inducers (tretinoin); mTOR inhibitors, topoisomerase inhibitors (doxorubicin (adriamycin), amsacrine, camptothecin, daunorubicin, dactinomycin, eniposide, epirubicin, etoposide, idarubicin, irinotecan (CPT-11) and mitoxantrone, topotecan, innotecan), corticosteroids (cortisone, dexamethasone, hydrocortisone, methylpednisolone, prednisone, and prenisolone); growth factor signal transduction kinase inhibitors; mitochondrial dysfunction inducers and caspase activators; chromatin disruptors.

These chemotherapeutic agents are used by itself with an galectin inhibitor, or in combination. Many combinatorial therapies have been developed in prior art, including but not limited to those listed in Table 1.

**Table 1: Exemplary conventional combination cancer chemotherapy**

| **Name** | **Therapeutic agents** |
|---|---|
| ABV | Doxorubicin, Bleomycin, Vinblastine |
| ABVD | Doxorubicin, Bleomycin, Vinblastine, Dacarbazine |
| AC (Breast) | Doxorubicin, Cyclophosphamide |
| AC (Sarcoma) | Doxorubicin, Cisplatin |
| AC (Neuroblastoma) | Cyclophosphamide, Doxorubicin |
| ACE | Cyclophosphamide, Doxorubicin, Etoposide |
| ACe | Cyclophosphamide, Doxorubicin |
| AD | Doxorubicin, Dacarbazine |
| AP | Doxorubicin, Cisplatin |
| ARAC-DNR | Cytarabine, Daunorubicin |
| B-CAVe | Bleomycin, Lomustine, Doxorubicin, Vinblastine |
| BCVPP | Carmustine, Cyclophosphamide, Vinblastine, Procarbazine, Prednisone |
| BEACOPP | Bleomycin, Etoposide, Doxorubicin, Cyclophosphamide, Vincristine, Procarbazine, Prednisone, Filgrastim |
| BEP | Bleomycin, Etoposide, Cisplatin |
| BIP | Bleomycin, Cisplatin, Ifosfamide, Mesna |
| BOMP | Bleomycin, Vincristine, Cisplatin, Mitomycin |
| CA | Cytarabine, Asparaginase |
| CABO | Cisplatin, Methotrexate, Bleomycin, Vincristine |
| CAF | Cyclophosphamide, Doxorubicin, Fluorouracil |
| CAL-G | Cyclophosphamide, Daunorubicin, Vincristine, Prednisone, Asparaginase |
| CAMP | Cyclophosphamide, Doxorubicin, Methotrexate, Procarbazine |
| CAP | Cyclophosphamide, Doxorubicin, Cisplatin |
| CaT | Carboplatin, Paclitaxel |
| CAV | Cyclophosphamide, Doxorubicin, Vincristine |
| CAVE ADD | CAV and Etoposide |
| CA-VP16 | Cyclophosphamide, Doxorubicin, Etoposide |
| CC | Cyclophosphamide, Carboplatin |
| CDDP/VP-16 | Cisplatin, Etoposide |
| CEF | Cyclophosphamide, Epirubicin, Fluorouracil |
| CEPP(B) | Cyclophosphamide, Etoposide, Prednisone, with or without/ Bleomycin |
| CEV | Cyclophosphamide, Etoposide, Vincristine |
| CF | Cisplatin, Fluorouracil or Carboplatin Fluorouracil |
| CHAP | Cyclophosphamide or Cyclophosphamide, Altretamine, Doxorubicin, Cisplatin |
| ChlVPP | Chlorambucil, Vinblastine, Procarbazine, Prednisone |
| CHOP | Cyclophosphamide, Doxorubicin, Vincristine, Prednisone |
| CHOP-BLBO | Add Bleomycin to CHOP |
| CISCA | Cyclophosphamide, Doxorubicin, Cisplatin |
| CLD-BOMP | Bleomycin, Cisplatin, Vincristine, Mitomycin |
| CMF | Methotrexate, Fluorouracil, Cyclophosphamide |
| CMFP | Cyclophosphamide, Methotrexate, Fluorouracil, Prednisone |
| CMFVP | Cyclophosphamide, Methotrexate, Fluorouracil, Vincristine, Prednisone |
| CMV | Cisplatin, Methotrexate, Vinblastine |
| CNF | Cyelophosphamide,Mitoxantrone, Fluorouracil |
| CNOP | Cyclophosphamide, Mitoxantrone, Vincristine, Prednisone |
| COB | Cisplatin, Vincristine, Bleomycin |
| CODE | Cisplatin, Vincristine, Doxorubicin, Etoposide |
| COMLA | Cyclophosphamide, Vincristine, Methotrexate, Leucovorin, Cytarabine |
| COMP | Cyclophosphamide, Vincristine, Methotrexate, Prednisone |
| Cooper Regimen | Cyclophosphamide, Methotrexate, Fluorouracil, Vincristine, Prednisone |
| COP | Cyclophosphamide, Vincristine, Prednisone |
| COPE | Cyclophosphamide, Vincristine, Cisplatin, Etoposide |
| COPP | Cyclophosphamide, Vincristine, Procarbazine, Prednisone |
| CP(Chronic lymphocytic leukemia) | Chlorambucil, Prednisone |
| CP (Ovarian Cancer) | Cyclophosphamide, Cisplatin |
| CT | Cisplatin, Paclitaxel |
| CVD | Cisplatin, Vinblastine, Dacarbazine |
| CVI | Carboplatin, Etoposide, Ifosfamide, Mesna |
| CVP | Cyclophosphamide, Vincristine, Prednisome |
| CVPP | Lomustine, Procarbazine, Prednisone |
| CYVADIC | Cyclophosphamide, Vincristine, Doxorubicin, Dacarbazine |
| DA | Daunorubicin, Cytarabine |
| DAT | Daunorubicin, Cytarabine, Thioguanine |
| DAV | Daunorubicin, Cytarabine, Etoposide |
| DCT | Daunorubicin, Cytarabine, Thioguanine |
| DHAP | Cisplatin, Cytarabine, Dexamethasone |
| DI | Doxorubicin, Ifosfamide |
| DTIC/Tamoxifen | Dacarbazine, Tamoxifen |
| DVP | Daunorubicin, Vincristine, Prednisone |
| EAP | Etoposide, Doxorubicin, Cisplatin |
| EC | Etoposide, Carboplatin |
| EFP | Etoposie, Fluorouracil, Cisplatin |
| ELF | Etoposide, Leucovorin, Fluorouracil, |
| EMA 86 | Mitoxantrone, Etoposide, Cytarabine |
| EP | Etoposide, Cisplatin |
| EVA | Etoposide, Vinblastine |
| FAC | Fluorouracil, Doxorubicin, Cyclophosphamide |
| FAM | Fluorouracil, Doxorubicin, Mitomycin |
| FAMTX | Methotrexate, Leucovorin, Doxorubicin |
| FAP | Fluorouracil, Doxorubicin, Cisplatin |
| F-CL | Fluorouracil Leucovorin |
| FEC | Fluorouracil, Cyclophosphamide, Epirubicin |
| FED | Fluorouracil, Etoposide, Cisplatin |
| FL | Flutamide, Leuprolide |
| FZ | Flutamide, Goserelin acetate implant |
| HDMTX | Methotrexate, Leucovorin |
| Hexa-CAF | Altretamine, Cyclophosphamide, Methotrexate, Fluorouracil |
| ICE-T | Ifosfamide, Carboplatin, Etoposide, Paclitaxel, Mesna |
| IDMTX/6-MP | Methotrexate, Mercaptopurine, Leucovorin |
| IE | Ifosfamide, Etoposie, Mesna |
| IfoVP | Ifosfamide, Etoposide, Mesna |
| IPA | Ifosfamide, Cisplatin, Doxorubicin |
| M-2 | Vincristine, Carmustine, Cyclophosphamide, Prednisone, Melphalan |
| MAC-III | Methotrexate, Leucovorin Dactinomycin, Cyclophosphamide |
| MACC | Methotrexate, Doxorubicin, Cyclophosphamide, Lomustine |
| MACOP-B | Methotrexate,.Leucovorin, Doxorubicin, Cyclophosphamide, Vincristine, Bleomycin, Prednisone |
| MAID | Mesna, Doxorubicin, Ifosfamide, Dacarbazine |
| m-BACOD | Bleomycin, Doxorubicin, Cyclophosphamide, Vincristine, Dexamethasone, Methotrexate, Leucovorin |
| MBC | Methotrexate, Bleomycin, Cisplatin |
| MC | Mitaxantrone, Cytarabine |
| MF | Methotrexate, Fluorouracil, Leucovorin |
| MICE | Ifosfamide, Carboplatin, Etoposide, Mesna |
| MINE | Mesna, Ifosfamide, Mitoxantrone, Etoposide |
| mini-BEAM | Carmustine, Etoposide, Cytarabine, Melphalan |
| MOBP | Bleomycin, Vincristine, Cisplatin, Mitomycin |
| MOP | Mechlorethamine, Vincristine, Procarbazine |
| MOPP | Mechlorethamine, Vincristine, Procarbazine, Prednisone |
| MOPP/ABV | Mechlorethamine, Vincristine, Procarbazine, Prednisone, Doxorubicin, Bleomycin, Vinblastine |
| MP (multiple myeloma) | Melphalan, Prednisone |
| MP (prostate cancer) | Mitoxantrone, Prednisone |
| MTX76-MO | Methotrexate, Mercaptopurine- . |
| MTX/6-MP/VP | Methotrexate, Mercaptopurine, Vincristine, Prednisone |
| MTX-CDDPAdr | Methotrexate, Leucovorin, Cisplatin, Doxorubicin |
| MV (breast cancer) | Mitomycin, Vinblastine |
| MV (acute myelocytic leukemia) | Mitoxantrone, Etoposide |
| M-VAC Methotrexate | Vinblastine, Doxorubicin, Cisplatin |
| MVP Mitomycin | Vinblastine, Cisplatin |
| MVPP | Mechlorethamine, Vinblastine, Procarbazine, Prednisone |
| NFL | Mitoxantrone, Fluorouracil, Leucovorin |
| NOVP | Mitoxantrone, Vinblastine, Vincristine |
| OPA | Vincristine, Prednisone, Doxorubicin |
| OPPA | Add Procarbazine to OPA. |
| PAC | Cisplatin, Doxozubicin |
| PAC-I | Cisplatin, Doxorubicin, Cyclophosphamide |
| PA CX | Cisplatin, Doxorubicin |
| PC | Paclitaxel, Carboplatin or Paclitaxel, Cisplatin |
| PCV | Lomustine, Procarbazine, Vincristine |
| PE | Paclitaxel, Estramustine |
| PFL | Cisplatin, Fluorouracil, Leucovorin |
| POC | Prednisone, Vincristine, Lomustine |
| ProMACE | Prednisone, Methotrexate, Leucovorin, Doxorubicin, Cyclophosphamide, Etoposide |
| ProMACE/cytaBOM | Prednisone, Doxorubicin, Cyclophosphamide, Etoposide, Cytarabine, Bleomycin, Vincristine, Methotroxate, Leucovorin, Cotrimoxazole |
| PRoMACB/MOPP | Prednisone, Doxorubicin, Cyclophosphamide, Etoposide, Mechlorethamine, Vincristine, Procarbazine, Methotrexate, Leucovorin |
| Pt/VM | Cisplatin, Teniposide |
| PVA | Prednisone, Vincristine, Asparaginase |
| PVB | Cisplatin, Vinblastine, Bleomycin |
| PVDA | Prednisone, Vincristine, Daunorubicin, Asparaginase |
| SMF | Streptozocin, Mitomycin, Fluorouracil |
| TAD | Mechlorethamine, Doxorubicin, Vinblastine, Vincristine, Bleomycin, Etoposide, Prednisone |
| TCF | Paclitaxel, Cisplatin, Fluorouracil |
| TIP | Paclitaxel, Ifosfamide, Mesna, Cisplatin |
| TTT | Methotrexate, Cytarabinc, Hydrocortisone |
| Topo/CTX | Cyclophosphamide, Topotecan, Mesna |
| VAB-6 | Cyclophosphamide, Dactinomycin, Vinblastine, Cisplatin, Bleomycin |
| VAC | Vincristine, Dactinomycin, Cyclophosphamide |
| VACAdr | Vincristine, Cyclophosphamide, Doxorubicin, Dactinomycin, Vincristine |
| VAD | Vincristine, Doxorubicin, Dexamethasone |
| VATH | Vinblastine, Doxorubicin, Thiotepa, Flouxymesterone |
| VBAP | Vincristine, Carmustine, Doxorubicin, Prednisone |
| VBCMP | Vincristine, Carmustine, Melphalan, Cyclophosphamide, Prednisone |
| VC | Vinorelbine, Cisplatin |
| VCAP | Vincristine, Cyclophosphamide, Doxorubicin, Prednisone |
| VD | Vinorelbine, Doxorubicin |
| VeIP | Vinblastine, Cisplatin, Ifosfamide, Mesna |
| VIP | Etoposide, Cisplatin, Ifosfamide, Mesna |
| VM | Mitomycin, Vinblastine |
| VMCP | Vincristine, Melphalan, Cyclophosphamide, Prednisone |
| VP | Etoposide, Cisplatin |
| V-TAD | Etoposide, Thioguanine, Daunorubicin, Cytarabine |
| 5 + 2 | Cytarabine, Daunorubicin, Mitoxantrone |
| 7+3 | Cytarabine with/, Daunorubicin or Idarubicin or Mitoxantrone |
| "8 in 1" | Methylprednisolone, Vincristine, Lomustine, Procarbazine, Hydroxyurea, Cisplatin, Cytarabine, Dacarbazine |

In addition to conventional chemotherapeutics, the agent of the subject method can also be compounds and antisense RNA, RNAi or other polynucleotides to inhibit the expression of the cellular components that contribute to unwanted cellular proliferation that are targets of conventional chemotherapy. Such targets arc, merely to illustrate, growth factors, growth factor receptors, cell cycle regulatory proteins, transcription factors, or signal transduction kinases.

The method of present invention is advantageous over combination therapies known in the art because it allows conventional chemotherapeutic agent to exert greater effect at lower dosage. In preferred embodiment of the present invention, the effective dose (ED₅₀) for a chemotherapeutic agent or combination of conventional chemotherapeutic agents when used in combination with galectin-3 inhibitor is at least 5 fold less than the ED₅₀ for the chemotherapeutic agent alone. Conversely, the therapeutic index (TT) for such chemotherapeutic agent or combination of such chemotherapeutic agent when used in combination with a galectin-3 inhibitor is at least 5 fold greater than the TI for conventional chemotherapeutic regimen alone.

### C. Other treatment methods

In yet other embodiments, the subject method combines a galectin-3 inhibitor with radiation therapies, including ionizing radiation, gamma radiation, or particle beams.

### D. Administration

A galectin-3 inhibitor or combination therapeutics containing a galectin-3 inhibitor may be administered orally, parenterally by intravenous injection, transdermally, by pulmonary inhalation, by intravaginal or intrarectal insertion, by subcutaneous implantation, intramuscular injection or by injection directly into an affected tissue, as for example by injection into a tumor site. In some instances the materials may be applied topically at the time surgery is carried out. In another instance the topical administration may be ophthalmic, with direct application of the therapeutic composition to the eye.

The materials are formulated to suit the desired route of administration. The formulation may comprise suitable excipients include pharmaceutically acceptable buffers, stabilizers, local anesthetics, and the like that are well known in the art. For parenteral administration, an exemplary formulation may be a sterile solution or suspension; For oral dosage, a syrup, tablet or palatable solution; for topical application, a lotion, cream, spray or ointment; for administration by. inhalation, a microcrystalline powder or a solution suitable for nebulization; for intravaginal or intrarectal administration, pessaries, suppositories, creams or foams. Preferably, the route of administration is parenteral more preferably intravenous.

### E. Exemplary Targets for Treatment

Galectin-3 inhibitors inhibit the growth of: a pancreatic tumor cell, a lung tumor cell, a prostate tumor cell, a breast tumor cell, a colon tumor cell, a liver tumor cell, a brain tumor cell, a kidney tumor cell, a skin tumor cell and an ovarian tumor cell, and therefore inhibit the growth of squamous cell carcinoma, a non-squamous cell carcinoma, a glioblastoma, a sarcoma, an adenocarcinoma, a melanoma, a papilloma, a neuroblastoma and leukemia.

The method of present invention is effective in treatment of various types of cell proliferative disorders and cancers, including but not limited to: psoriasis, rheumatoid arthritis, lamellar ichthyosis, epidermolytic hyperkeratosis, restenosis, endometriosis, benign hyperplasias, diseases associated with corneal neovascularization, or abnormal wound healing, and various types of cancer, including renal cell cancer, Kaposi's sarcoma, chronic lymphocytic leukemia, lymphoma, mesothelioma, breast cancer, sarcoma, ovarian carcinoma, rectal cancer, throat cancer, melanoma, colon cancer, bladder cancer, mastocytoma, lung cancer, liver cancer, mammary adenocarcinoma, pharyngeal squamous cell carcinoma, prostate cancer, pancreatic cancer, gastrointestinal cancer, or stomach cancer. The method is also effective in preventing angiogenesis associated with neoplastic growth or treating diseases associated with chronic inflammation, and autoimmune diseases.

Further disclosure of related compositions and use are described in U.S. Patent 6,680,306 and U.S. Patent Application Serial Nos. 08/024,487, 10/299,478, 10/176,022, and 60/461,006 the disclosures of which are incorporated herein by reference.

Of course, the method of present invention is more effective and is preferred if the targeted cancer cells have elevated levels or active galactin-3 involved in malignant proliferation of the tumors and non-solid neoplasm. Therefore, it is beneficial to determine the expression level and phosphorylation state of galectin-3, as well as determine the intercellular locations of galectin-3.

The presence of galectin-3 in a tumor can be determined by immunodetection using antibodies specific to galectin-3, either through enzyme-linked immunosorbent assays, or immunohistochemistry of solid tumor samples. The immunohistochemistry will also allow determination of the intracellular localization of galectin-3 in a tumor sample. By using monoclonal antibodies specific to phosphorylated galectin-3, the phosphorylation state of galectin-3 can also be determined by the same techniques. Galectin-3 expression can be determined by detecting galectin-3 mRNA in Southern blots, using probes specific to a galectin-3 nucleotide sequence. Alternatively, quantitative polymerase chain reaction may be done, using a pair of primers specific to galectin-3 gene. Once the expression level and the status of galectin-3 are determined, a patient with cancerous growth which have elevated levels of galectin-3 activities are treated with galectin-3 inhibitors along with other anti-cancer therapies as necessary.

Because galectin-3 and Bcl-2 or Bcl-xL interact and because galectin-3 inhibitors are especially useful to treat cells with elevated Bcl-2 or Bcl-xL activities, it is beneficial to determine the level of active Bcl-2 and Bcl-xL in a tumor or in leukemic cells in a patient. Bcl-2 or Bcl-xL can be detected using the same techniques as described above for galectin-3, except that specific probes and antibodies to detect the appropriate proteins are used.

### F. Examples

### Example 1. Promotion of apoptosis by a modified pectin

Experiments were performed to demonstrate the ability of a modified pectin to promote apoptosis in a cell line with high Bcl-2 expression and chemoresistance.

Cell line DoHH2 is a spontaneously growing EBV-negative B-cell line, established from the pleural fluid cells of a patient with centroblastic/centrocytic non-Hodgkin's lymphoma, that had transformed into an immunoblastic lymphoma. Kluin-Nelemans et al., "A new non-Hodgkin's B-cell line (DoHH2) with a chromosomal translocation t(14;18)(q32;q21)," Leukemia 1991 Mar;5(3):221-4. The expression of Bcl-2 is upregulated in DoHH2 due to chromosomal translocation, and the cell line is known to have high chemoresistance that is dependent on the status of Bcl-2.

When treated with a Bcl-2 antisense polynucleotide, DoHH2 proceeds to apoptosis, indicating the overexpression of Bcl-2 is a cause of lack of apoptosis.

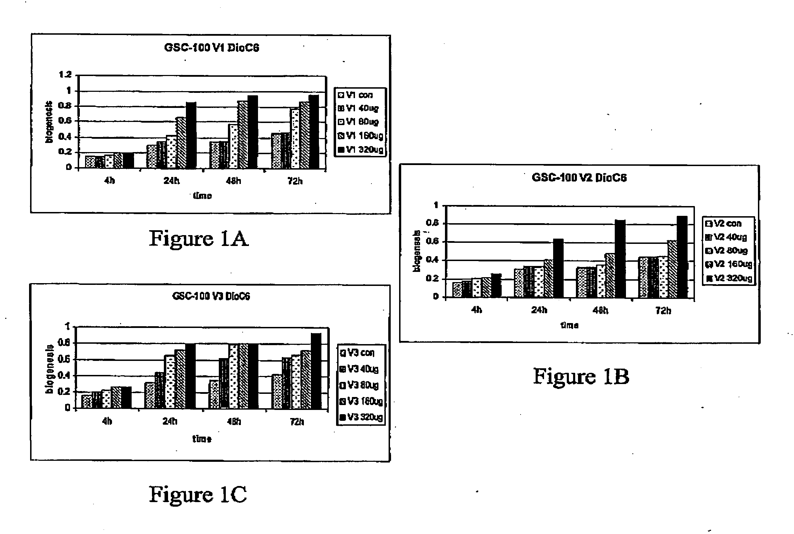

DoHH2 cells were exposed to modified pectin GCS-100 in three different formulations, V1, V2, and V3. Formulation V1 contained 12.6% ethanol, V2 contained 15% ethanol, and V3 contained 0.2% ethanol. *In vitro* apoptosis was quantitated by DioC6(3) stain as a measure of mitochondrial depolarization at 4, 24, 48, and 72 hours after 0, 40, 80, 160, or 320 µg /ml of each formulation was added to cell culture. See Figures 1A -1C. All samples demonstrated increased apoptosis over time, but the addition of GCS-100 increased the number of cells undergoing apoptosis in a dose-dependent manner. The three formulations performed similarly at the highest dose of 320 µg /ml, but at lower dosages of 40, 80, or 160 µg /ml, formulation V3, which contained the least amount of ethanol, was more effective in inducing apoptosis at earlier time points compared to formulation V1 or V2.

### Example 2. Enhancement of efficacy of Etoposide by GCS-100

Etoposide (4'-demethylepipodophyllotoxin 9-(4,6-o-ethylidene- beta-D-glucopyranoside)), a.k.a. VP-16, is a cytotoxic chemotherapeutic which inhibits topoisomerase II by inducing the formation of and stabilizing a cleavable enzyme-DNA complex. Experiments were performed to demonstrate modified pectin GCS-100's ability to enhance the cytotoxic effects of etoposide in an *in vitro* cell culture system.

DoHH2 cells, as described in Example 1, were cultured in RPMI1640 medium and exposed to otoposide at various concentrations for 24 hours in the presence or absence of 40 µg/ml of GCS-100. The formulation of GCS-100 used was V3, described in Example 1. *In vitro* apoptosis was quantitated by DioC6(3) stain as a measure of mitochondrial depolarization after 24 hour exposure to the combination of etoposide and GCS-100. The ability of GCS-100 to enhance apoptosis was tested at five concentrations of etoposide within a 25-fold range.

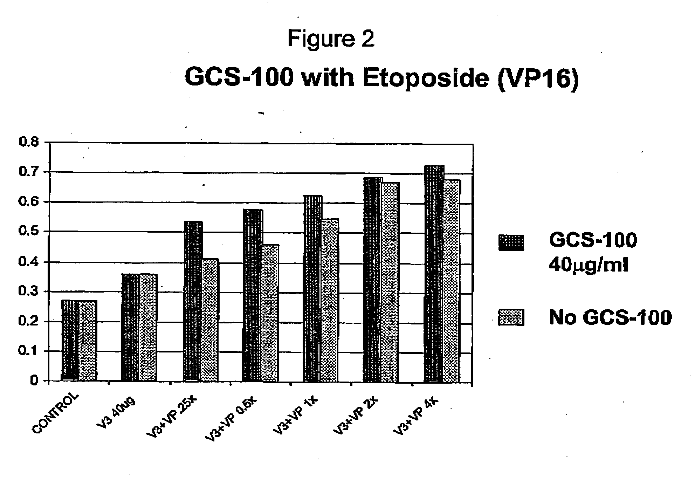

As shown in Figure 2, GCS-100 enhanced the etoposide-induced apoptosis in a statistically significant manner at lower etoposide concentrations.

The foregoing discussion has been primary directed toward modified pectin materials and materials which interact with galectin-3; however, it is to be understood that other galectins are also known to be involved in the progress of various cancers, and both the modified pectin material as well as the other therapeutic materials discussed hereinabove interact with galectins. Therefore, other materials may be employed in the practice of the present invention. The foregoing discussion and description is illustrative of specific embodiments, but is not meant to be a limitation upon the practice thereof It is the following claims, including all equivalents, which define the scope of the invention.
1. A method for enhancing the efficacy of a therapeutic treatment for proliferative disorders in a patient wherein cytotoxicity of said therapeutic treatment is influenced by the status of an anti-apoptotic Bcl-2 protein of a cancer cell or cell undergoing unwanted proliferation in the patient, said method comprising a therapeutic regimen including conjointly administering to the patient said therapeutic treatment and a therapeutically effective amount of an agent that inhibits galectin-3 activity ("galectin-3 inhibitor").
2. A method for reducing the rate of growth of a tumor cell or a cell undergoing unwanted proliferation in a patient, wherein said method comprises administering to the patient a therapeutic regimen comprising:
   (i) a chemotherapeutic agent whose cytotoxicity is influenced by the status of an anti-apoptotic Bcl-2 protein for said cell, and
   (ii) a galectin-3 inhibitor in an amount sufficient to reduce the levels of one or more G1/S cyclins in said cell.
3. A method for reducing the rate of growth of a tumor cell or a cell undergoing unwanted proliferation which expresses galectin-3 in a patient, said method comprising:
   (i) obtaining a sample of said cell from a patient;
   (ii) ascertaining the galectin-3 status of the cell sample; and
   (iii) for a patient having a cell sample that expresses galectin-3, administering a therapeutic regimen including a galectin-3 inhibitor in an amount sufficient to reduce the levels of one or more G1/S cyclins in said cell.
4. A method for enhancing the pro-apoptotic effect of a chemotherapeutic agent that interferes with DNA replication fidelity or cell-cycle progression of a cancer cell or a cell undergoing unwanted proliferation in a patient, said method comprising therapeutic regimen including conjointly administering to said patient said chemotherapeutic agent and a galectin-3 inhibitor in an amount sufficient to reduce the levels of one or more G1/S cyclins in the cell.
5. A method for reducing the rate of growth of a tumor cell or a cell undergoing unwanted proliferation which expresses an anti-apoptotic Bcl-2 protein, comprising:
   (i) obtaining a sample of said cell from a patient;
   (ii) ascertaining the anti-apoptotic Bcl-2 protein status of the cell sample; and
   (iii) for a patient having a cell sample expressing a wild-type or elevated level or Bcl-2 proteins, administering to said patient a therapeutic regimen including a therapeutically effective amount of a galectin-3 inhibitor.
6. The method of clause 5 wherein said tumor is chemoresistant.
7. The method of clause 2, wherein said chemotherapeutic agent is cytostatic when administered in the absence of said galectin-3 inhibitor but is rendered cytotoxic when administered conjointly with said galectin-3 inhibitor.
8. The method of any of 1, 2 or 5, wherein the therapeutic regimen includes a chemotherapeutic agent that is influenced by the Bcl-2 or Bcl-xL status of said cell for cytotoxicity.
9. The method of any oMatBM-1 to 5, wherein said galectin-3 inhibitor inhibits signal transduction by galectin-3 and binds to galectin-3 with a Kd of 10⁻⁶M or less.
10. The method of clause 9, wherein said galectin-3 inhibitor is a carbohydrate.
11. The method of clause 9, wherein said galectin-3 inhibitor is an antibody.
12. The method of clause 9, wherein said galectin-3 inhibitor is a small molecule.
13. The method of clause 9, wherein said galectin-3 inhibitor is a peptide or polypeptide.
14. The method of any of clauses 1 to 5, wherein said galectin-3 inhibitor inhibits interaction of galectin-3 with Bcl-2.
15. The method of any of clauses 1 to 5, wherein said galectin-3 inhibitor inhibits phosphorylation of galectin-3.
16. The method of clause 15, wherein said galectin-3 inhibitor inhibits phosphorylation of galectin-3 at Ser-6.
17. The method of any of clauses 1 to 5, wherein said galectin-3 inhibitor inhibits translocation of galectin-3 between the nucleus and cytoplasm or inhibits galectin-3 translocation to the perinuclear membranes.
18. The method of any of clauses 1 to 5, wherein said galectin-3 inhibitor inhibits expression of galectin-3.
19. The method of clause 18, wherein said galectin-3 inhibitor is an antisense or RNAi construct having a sequence corresponding to a portion of the mRNA sequence transcribed from the galectin-3 gene.
20. The method of any of clauses 1, 2, or 4, wherein the chemotherapeutic agent induces mitochondrial dysfunction and/or caspase activation.
21. The method of any of clauses 1, 2, 4, or 7, wherein the chemotherapeutic agent induces cell cycle arrest at G2/M in the absence of said galectin-3 inhibitor.
22. The method of any of clauses 1, 2, or 4, wherein said chemotherapeutic agent is an inhibitor of chromatin function.
23. The method of clause 22, wherein said chemotherapeutic agent is a DNA topoisomerase inhibitor.
24. The method of clause 23, wherein said topoisomerase inhibitor is selected from adriamycin, amsacrine, camptothecin, daunorubicin, dactinomycin, doxorubicin, eniposide, epirubicin, etoposide, idarubicin, irinotecan (CPT-11) and mitoxantrone.
25. The method of clause 22, wherein said chemotherapeutic agent is a microtubule inhibiting drug.
26. The method of clause 25, wherein said microtubule inhibiting drug is a taxane.
27. The method of clause 26, wherein said microtubule inhibiting drug is selected from paclitaxel, docetaxel, vincristin, vinblastin, nocodazole, epothilones and navelbine.
28. The method of any of clauses 1, 2, or 4, wherein said chemotherapeutic agent is a DNA damaging agent.
29. The method of clause 28, wherein said DNA, damaging agent is selected from actinomycin, amsacrine, anthracyclines, bleomycin, busulfan, camptothecin, carboplatin, chlorambucil, cisplatin, cyclophosphamide, cytoxan, dactinomycin, daunorubicin, docetaxel, doxorubicin, epirubicin, hexamethylmelamineoxaliplatin, iphosphamide, melphalan, merchlorehtamine, mitomycin, mitoxantrone, nitrosourea, plicamycin, procarbazine, taxol, taxotere, teniposide, methylenethiophosphoramide and etoposide (VP16).
30. The method of any of clauses 1, 2, or 4, wherein said chemotherapeutic agent is an antimetabolite.
31. The method of clause 30, wherein said antimetabolite is selected from folate antagonists, pyrimidine analogs, purine analogs, and sugar-modified analogs.
32. The method of any of clauses 2, or 4, wherein said chemotherapeutic agent is a DNA synthesis inhibitor.
33. The method of clause 32, wherein said DNA synthesis inhibitor is a thymidilate synthase inhibitors, such as 5-fluorouracil.
34. The method of clause 32, wherein said DNA synthesis inhibitor is a dihydrofolate reductase inhibitor, such as methoxtrexate.
35. The method of clause 32, wherein said DNA synthesis inhibitor is a DNA polymerase inhibitor, such as fludarabine.
36. The method of any of clauses 1, 2, or 4, wherein said chemotherapeutic agent is a DNA binding agent.
37. The method of clause 36, wherein said DNA binding agent is an intercalating agent.
38. The method of any of clauses 1, 2, or 4, wherein said chemotherapeutic agent is a DNA, repair inhibitor.
39. The method of clauses or 5, wherein the therapeutic regimen includes at least one additional chemotherapeutic agent that affects growth of the tumor cell in an additive or synergistic manner with said galectin-3 inhibitor.
40. The method of clause 39, wherein said additional chemotherapeutic agent is a corticosteroid.
41. The method of clause 40, wherein said corticosteroid is selected from cortisone, dexamethasone, hydrocortisone, methylprednisolone, prednisone, and prenisolone.
42. The method of clause 39, wherein said therapeutic regimen is a combinatorial therapy selected from ABV, ABVD, AC (Breast), AC (Sarcoma), AC (Neutoblastoma), ACE, ACe, AD, AP, ARAC-DNR, B-CAVe, BCVPP, BEACOPP, BEP, BIP, BOMP, CA, CABO, CAP, CAL-G, CAIVIP, CAP, CaT, CAV, CAVE ADD, CA-VP16, CC, CDDP/VP-16, CEF, CEPP(B), CEV, CF, CHAP, Ch1VPP, CHOP, CHOP-BLBO, CISCA, CLD-BOMP, CMF, CMFP, CMFVP, CMV, CNF, CNOP, COB, CODE, COMLA, COMP, Cooper Regimen, COP, COPE, COPP, CP - Chronic Lymphocytic Leukemia, CP - Ovarian Cancer, CT, CVD, CVI, CVP, CVPP, CYVADIC, DA, DAT, DAV, DCT, DHAP, DI, DTIC/Tamoxifen, DVP, EAP, EC, EFP, ELF, EMA 86, EP, LVA, FAC, PAM, FAMTX, FAP, F-CL, FEC, FED, FL, FZ, HDMTX, Hexa-CAF, ICE-T, IDMTX/6-MP, IE, IfoVP, IPA, M-2, MAC-III, MACC, MACOP-B, MAID, m-BACOD, MBC, MC, MF, MICE, MINE, mini-BEAM, MOBP, MOP, MOPP, MOPP/ABV, MP - multiple myeloma, MP- prostate cancer, MTX/6-MO, MTX/6-MP/VP,MTX-CDDPAdr, MV - breast cancer, MV - acute myelocytic leukemia, M-VAC Methotrexate, MVP Mitomycin. MVPP, NFL, NOVP, OPA, OPPA, PAC, PAC-I, PA-CI, PC, PCV, PE, PFL, POC, ProMACE, ProMACE/cytaBOM, PRoMACE/MOPP, Pt/VM, PVA, PVB, PVDA, SMF, TAD, TCF, TIP, TTT, Topo/CTX, VAB-6, VAC, VACAdr, VAD, VATH, VBAP, VBCMP, VC, VCAP, VD, VelP, VIP, VM, VMCP, VP, V-TAD, 5 + 2,7 +3,"8 in 1".
43. The method of clause 39, wherein the additional chemotherapeutic agent is selected from aminoglutethimide, amsacrine, anastrozole, asparaginase, bcg, bicalutamide, bleomycin, buserelin, busulfan, campothecin, capecitabine, carboplatin, carmustine, chlorambucil, cisplatin, cladribine, clodronate, colchicine, cyclophosphamide, cyproterone, cytarabine, dacarbazine, dactinomycin, daunorubicin, dienestrol, diethylstilbestrol, docetaxel, doxorubicin, epirubicin, estradiol, estramustine, etoposide, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, ironotecan, letrozole, leucovorin, leuprolide, levamisole, lomustine, mechlorethamine, medroxyprogesterone, megestrol, melphalan, mercaptopurine, mesna, methotrexate, mitomycin, mitotane, mitoxantrone, nilutamide, nocodazole, octreotide, oxaliplatin, paclitaxel, pamidronate, pentostatin, plicamycin, porfimer, procarbazine, raltitrexed, rituximab, streptozocin, suramin, tamoxifen, temozolomide, teniposide, testosterone, thioguanine, thiotepa, titanocene dichloride, topotecan, trastuzumab, tretinoin, vinblastine, vincristine, vindesine, and vinorelbine.
44. The method of any of clauses 1, 2, 4, or 7, wherein said chemotherapeutic agent is selected from tamoxifen, 4-(3-chloro-4-fluorophenylamino)-7-methoxy-6-(3-(4-α-merpholinyl)propoxy)quinazoline, 4-(3-ethynylphenylamino)-6,7-bis(2-methoxyethoxy)quinazoline, hormones, steroids, steroid synthetic analogs, 17a-ethinylestradiol, diethylstilbestrol, testosterone, prednisone, fluoxymesterone, dromostanolone propionate, testolactone, megestrolacetate, methylprednisolone, methyl-testosterone, prednisolone, triamcinolone, chlorotrianisene, hydroxyprogesterone, aminoglutethimide, estramustine, medroxyprogesteroneacetate, leuprolide, flutamide, toremifene, Zoladex, antiangiogenics, matrix metalloproteinase inhibitors, VEGF inhibitors, ZD6474, SU6668, SU11248, anti-Her-2 antibodies (ZD1839 and OSI774), EGFR inhibitors, EKB-569, Imclone antibody C225, src inhibitors, bicalutamide, epidermal growth factor inhibitors, Her-2 inhibitors, MEK-1 kinase inhibitors, MAPK kinase inhibitors, P13 inhibitors, PDGF inhibitors, combretastatins, MET kinase inhibitors, MAP kinase inhibitors, inhibitors of non-receptor and receptor tyrosine kinases (imatinib), inhibitors of integrin signaling, and inhibitors of insulin-like growth factor receptors.
45. The method of clauses 1, 3, or 5, wherein said therapeutic regimen includes ionizing radiation.
46. The method of any of clauses 1 to 5, used to inhibit growth of a tumor cell selected from a pancreatic tumor cell, lung tumor cell, a prostate tumor cell, a breast tumor cell, a colon tumor cell, a liver tumor cell, a brain tumor cell, a kidney tumor cell, a skin tumor cell, an ovarian tumor cell and a leukemic blood cell.
47. The method of any of clauses 1 to 5, used to inhibit growth of a tumor cell selected from squamous cell carcinoma, non-squamous cell carcinoma, glioblastoma, sarcoma, adenocarcinoma, melanoma, papilloma, neuroblastoma, myeloma, lymphoma, and leukemia.
48. The method of any of clauses 1 to 5, used in the treatment of a proliferative disorder selected from renal cell cancer, Kaposi's sarcoma, chronic lymphocytic leukemia, lymphoma, mesothelioma, breast cancer, sarcoma, ovarian carcinoma, rectal cancer, throat cancer, melanoma, colon cancer, bladder cancer, mastocytoma, lung cancer, liver cancer, mammary adenocarcinoma, pharyngeal squamous cell carcinoma, prostate cancer, pancreatic cancer, gastrointestinal cancer, and stomach cancer.
49. The method of any of clauses 1 to 5, used in the treatment of a proliferative disorder selected from chronic inflammation, psoriasis, endometriosis, benign hyperplasias, or diseases associated with corneal neovascularization.
50. The method of any of clauses 1 to 5, wherein said galectin-3 inhibitor is a partially depolymerized pectin.
51. The method of clause 50, wherein said partially depolymerized pectin is a substantially demethoxylated polygalacturonic acid which is interrupted with rhamnose residues.
52. The method of clause 50, wherein said partially depolymerized pectin consists essentially of a homogalacturonan backbone and neutral sugar side chains having a low degree of branching dependent from the backbone.
53. The method of clause 50, wherein said partially depolymerized pectin comprises a pH modified pectin, an enzymatically modified pectin, and/or a thermally modified pectin.
54. The method of clause 50, wherein said partially depolymerized pectin comprises a modified citrus pectin.
55. The method of clause 50, wherein, said partially depolymerized pectin has a molecular weight of 1 to 500 kilodaltons (kDa).
56. The method of clause 50 wherein said partially depolymerized pectin has a molecular weight of 10 to 250 kDa.
57. The method of Clause 50 wherein said partially depolymerized pectin has a molecular weight of 50 to 200 kDa.
58. The method of Clause 50 wherein said partially depolymerized pectin has a molecular weight of 70 to 150 kDa.
59. The method of Clause 50 wherein said partially depolymerized pectin has a molecular weight of 80 to 100 kDa.
60. The method of Clause 50, wherein said partially depolymerized pectin comprises less than 5 percent ethanol.
61. The method of any one of clauses 1, 2, or 4, wherein the effective dose (ED₅₀) for said therapeutic treatment or chemotherapeutic agent when used in combination with said galectin-3 inhibitor is at least 5 fold less than the ED₅₀ for said therapeutic treatment or chemotherapeutic agent alone.
62. The method of any one of clauses 1, 2, or 4, wherein the therapeutic index (TT) for said therapeutic treatment or chemotherapeutic agent when used in combination with said galectin-3 inhibitor is at least 5 fold greater than the TI for said chemotherapeutic agent alone.
63. The method of any one of clauses 1,2 or 4, wherein said galectin-3 inhibitor is administered simultaneously with said therapeutic treatment.
64. The method of any one of clauses 1, 2, or 4, wherein said galectin-3 inhibitor is administered before administering said therapeutic treatment.
65. The method of any one of clauses 1, 2, or 4, wherein said galectin-3 inhibitor is administered after administering said therapeutic treatment.
66. The method of any one of clauses 1 to 5, wherein said galectin-3 inhibitor is administered parenterally.
67. The method of clause 66, wherein said galectin-3 inhibitor is administered by intravenous infusion.
68. The method of any one of clauses 1 to 5, wherein said galectin-3 inhibitor is administered orally.
69. The method of any one of clauses 1 to 5, wherein said galectin-3 inhibitor is administered by inhalation.
70. The method of any one of clauses 1 to 5, wherein said galectin-3 inhibitor is administered by topically.
71. The method of any one of clauses 1 to 5, wherein said galectin-3 inhibitor is administered by subcutaneous injection.
72. The method of any one of clauses 1 to 5, wherein said galectin-3 inhibitor is administered by intramuscular or intraperitoneal injection or infusion.
73. A kit comprising (i) a chemotherapeutic agent that interferes with DNA replication fidelity or cell-cycle progression of cells undergoing unwanted proliferation, (ii) a therapeutically effective amount of a galectin-3 inhibitor; and (iii) instructions and/or a label for conjoint administration of the chemotherapeutic agent and the galectin-3 inhibitor.
74. A packaged pharmaceutical comprising (i) a therapeutically effective amount of a galectin-3 inhibitor, and (ii) instructions and/or a label for administration of the galectin-3 inhibitor for the treatment of patients having tumors that that express galectin-3.

## Claims

1. Use of a therapeutically effective amount of an agent that inhibits galectin-3 activity ("galectin-3 inhibitor") for the manufacture of a medicament for enhancing the efficacy of a therapeutic treatment such as administration of a chemotherapeutic agent, radiation therapy, or surgery for proliferative disorders in a patient, wherein cytotoxicity of said therapeutic treatment is influenced by the status of an anti-apoptotic Bcl-2 protein of a cancer cell or cell undergoing unwanted proliferation in the patient and wherein said medicament is to be administered conjointly to the patient with said therapeutic treatment.

2. Use of a (i) a chemotherapeutic agent whose cytotoxicity is influenced by the status of an anti-apoptotic Bcl-2 protein for said cell, and (ii) a galcctin-3 inhibitor in an amount sufficient to reduce the levels of one or more G1/S cyclins in said cell for the manufacture of a medicament for reducing the rate of growth of a tumour cell or a cell undergoing unwanted proliferation in a patient.

3. Use of a chemotherapeutic agent that interferes with DNA replication fidelity or cell-cycle progression of a cancer cell or a cell undergoing unwanted proliferation in a patient and a galectin-3 inhibitor reducing the levels of one or more G1/S cyclins in the cell for the manufacture of a medicament for enhancing the pro-apoptotic effect of the chemotherapeutic agent.

4. The use of claim 1, wherein said galectin-3 inhibitor is a carbohydrate, antibody, small molecule, peptide or polypeptide.

5. The use of any of claims 1 - 3, wherein the chemotherapeutic agent is an anti-angiogenic agent or an apoptosis inducer.

6. The use of any of claims 1-3, wherein the chemotherapeutic agent is selected from an antibody; a DNA damaging agent; an antiproliferative agent; an antimitotic agent; a VEGF inhibitor; a receptor tyrosine kinase inhibitor; an mTOR inhibitor; a growth factor signal transduction kinase inhibitor; and a combination of cyclophosphamide, doxorubicin, vincristine and prednisone.

7. The use of any of claims 1 - 3, wherein the medicament includes at least one additional chemotherapeutic agent that affects growth of the tumour cell in an additive or synergistic manner with said galcctin-3 inhibitor.

8. The use of claim 7, wherein said additional chemotherapeutic agent is a corticosteroid.

9. The use of claim 8, wherein said corticosteroid is selected from cortisone, dexamethasone, hydrocortisone, methylprednisolone, prednisone and prenisolonc.

10. The use of any of claims 1 - 3 to inhibit growth of a tumour cell selected from a pancreatic tumour cell, lung tumour cell, a prostate tumour cell, a breast tumour cell, a colon tumour cell, a liver tumour cell, a brain tumour cell, a kidney tumour cell, a skin tumour cell, an ovarian tumour cell and a leukemic blood cell and from cells of squamous cell carcinoma, non-squamous cell carcinoma, glioblastoma, sarcoma, adenocarcinoma, melanoma, papilloma, neuroblastoma, myeloma, lymphoma and leukaemia or in the treatment of a proliferative disorder selected from renal cell cancer, Kaposi's sarcoma, chronic lymphocytic leukaemia, lymphoma, mesothelioma, breast cancer, sarcoma, ovarian carcinoma, rectal cancer, throat cancer, melanoma, colon cancer, bladder cancer, mastocytoma, lung cancer, liver cancer, mammary adenocarcinoma, pharyngeal squamous cell carcinoma, prostate cancer, pancreatic cancer, gastrointestinal cancer, stomach cancer, chronic inflammation, psoriasis, endometriosis, benign hyperplasias, or disease associated with corneal neovascularisation.

11. The use of any of claims 1- 3, wherein said galectin-3 inhibitor is a partially depolymerised pectin.

12. The use of claim 11, wherein said partially depolymerised pectin is a substantially demethoxylated polygalacturonic acid which is interrupted with rhamnose residues.

13. The use of claim 11, wherein said partially depolymerised pectin consists essentially of a homogalacturonan backbone and neutral sugar side chains having a low degree of branching dependent from the backbone.

14. The use of claim 11, wherein said partially depolymerised pectin has one or more of the following characteristics: (a) comprises a pH modified pectin, an enzymatically modified pectin, and/or a thermally modified pectin (b) comprises a modified citrus pectin; (c) has a molecular weight of 1 to 500 kilodaltons (kDa) and (d) comprises less than 5 percent ethanol.

15. A kit comprising (i) a chemotherapeutic agent that interferes with DNA replication fidelity or cell-cycle progression of cells undergoing unwanted proliferation, (ii) a therapeutically effective amount of a galectin-3 inhibitor; and (iii) instructions and/or a label for conjoint administration of the chemotherapeutic agent and the galectin-3 inhibitor.

16. A packaged pharmaceutical comprising (i) a therapeutically effective amount of a galectin-3 inhibitor; and (ii) instructions and/or a label for administration of the galectin-3 inhibitor for the treatment of patients having tumours that express galectin-3.
